# FASCICULE DE BREVET EUROPEEN

(11) **EP 2 791 328 B1**
(45) Date de publication et mention de la délivrance du brevet: **09.01.2019**
(21) Numéro de dépôt: 12813901.1
(22) Date de dépôt: 27.11.2012
(51) Int. Cl.: C12P 21/00

(54) **PROCÉDÉ DE PRODUCTION D'UN COCKTAIL ENZYMATIQUE UTILISANT LES RÉSIDUS SOLIDES D'UN PROCÉDÉ DE CONVERSION BIOCHIMIQUE DE MATÉRIAUX LIGNO-CELLULOSIQUES**
VERFAHREN ZUR HERSTELLUNG EINES ENZYMCOCKTAILS MIT DER FESTEN RÜCKSTÄNDEN AUS VERFAHREN ZUR BIOCHEMISCHEN UMWANDLUNG VON LIGNOCELLULOSEMATERIALIEN
METHOD FOR PRODUCING AN ENZYME COCKTAIL USING THE SOLID RESIDUES FROM A PROCESS FOR BIOCHEMICALLY CONVERTING OF LIGNOCELLULOSIC MATERIALS

(30) Priorité: 14.12.2011 FR 1103857
(43) Date de publication de la demande: 22.10.2014
(73) Titulaire: IFP Energies nouvelles, 92500 Rueil-Malmaison (FR)
(72) Inventeur: BEN CHAABANE, Fadhel, F-75020 Paris (FR); LOURET, Sylvain, F-69003 Lyon (FR)
(74) Mandataire: IFP Energies nouvelles
(86) Numéro de dépôt international: PCT/FR2012/000489
(87) Numéro de publication internationale: WO 2013/087998

(56) Documents cités:
- EP-A1- 1 690 944
- EP-A1- 2 371 950
- PRACHAND SHRESTHA ET AL: "Enzyme Production by Wood-Rot and Soft-Rot Fungi Cultivated on Corn Fiber Followed by Simultaneous Saccharification and Fermentation", JOURNAL OF AGRICULTURAL AND FOOD CHEMISTRY, vol. 57, no. 10, 27 mai 2009 (2009-05-27), pages 4156-4161, XP055033353, ISSN: 0021-8561, DOI: 10.1021/jf900345n
- CHAHAL D S ET AL: "Solid-state fermentation with Trichoderma reesei for cellulase production", APPLIED AND ENVIRONMENTAL MICROBIOLOGY, AMERICAN SOCIETY FOR MICROBIOLOGY, US, vol. 49, no. 1, 1985, pages 205-210, XP003016095, ISSN: 0099-2240
- CHAHAL P S ET AL: "PRODUCTION OF CELLULASE IN SOLID-STATE FERMENTATION WITH TRICHODERMA REESEI MCG 80 ON WHEAT STRAW", APPLIED BIOCHEMISTRY AND BIOTECHNOLOGY, HUMANA PRESS, INC, UNITED STATES, vol. 57/58, 1996, pages 433-442, XP008054568, ISSN: 0273-2289, DOI: 10.1007/BF02941724

## Description

### DOMAINE DE L'INVENTION

La présente invention concerne la production des enzymes cellulolytiques et hémicellulolytiques, notamment dans le cadre de la production d'éthanol à partir de matériaux cellulosiques ou ligno-cellulosiques.

### ART ANTÉRIEUR

Depuis les années 1970, la transformation de matériaux ligno-cellulosique en éthanol, après hydrolyse des polysaccharides constitutifs en sucres fermentescibles, a fait l'objet de très nombreux travaux. On peut citer par exemple les travaux de référence du National Renewable Energy Laboratory (Process Design and Economics for Biochemical Conversion of Lignocellulosic Biomass to Ethanol, Humbird et al., NREL/ TP-5100-57764, May 2011).

Les matériaux ligno-cellulosiques sont des matériaux cellulosiques, c'est-à-dire constitués à plus de 90% poids de cellulose, et/ou lignocellulosique, c'est-à-dire consitués de cellulose, d'hémicelluloses, qui sont des polysaccharides essentiellement constitués de pentoses et d'hexoses et de lignine, qui est une macromolécule de structure complexe et de haut poids moléculaire, composée d'alcools aromatiques reliés par des liaisons éther.

Le bois, les pailles, les rafles de maïs sont les matériaux lignocellulosiques les plus utilisés, mais d'autres ressources, cultures forestières dédiées, résidus de plantes alcooligènes, sucrières et céréalières, produits et résidus de l'industrie papetière et des produits de transformation des matériaux cellulosiques et ligno-cellulosiques sont utilisables. Ils sont constitués pour la plupart d'environ 35 à 50 % de cellulose, 20 à 30 % d'hémicellulose et de 15 à 25 % de lignine.

Le procédé de transformation des matériaux ligno-cellulosiques en éthanol comprend une étape de prétraitement physico-chimique, suivie d'une étape d'hydrolyse enzymatique ou chimique, d'une étape de fermentation éthanolique des sucres libérés, la fermentation éthanolique et l'hydrolyse enzymatique pouvant être conduites simultanément, et d'une étape de récupération de l'éthanol.

Le matériau prétraité est hydrolysé, soit par voie acide, soit par voie enzymatique avec l'utilisation d'enzymes cellulolytiques et/ou hémicellulolytiques.

La voie acide, effectuée à l'aide d'acide fort, acide sulfurique notamment, est efficace mais requiert d'importantes quantités de produits chimiques (acide puis base pour la neutralisation). L'hydrolyse enzymatique ne présente pas cet inconvénient ; elle s'effectue par ailleurs dans des conditions douces et est efficace. En revanche, le coût des enzymes reste très élevé, représentant de 30 à 50% du coût de transformation du matériau ligno-cellulosique en éthanol. De ce fait, beaucoup de travaux ont été conduits pour réduire ce coût : l'optimisation de la production d'enzymes d'abord, en sélectionnant les souches hyperproductrices et en améliorant les procédés de production desdites enzymes, la diminution de la quantité d'enzymes en hydrolyse ensuite, en optimisant la phase de prétraitement ou en améliorant l'activité spécifique de ces enzymes.

Cependant, la diminution de la quantité d'enzymes peut conduire à la non conversion d'une fraction importante de la cellulose, induisant la production d'une quantité importante de résidu solide à l'issue de l'étape d'hydrolyse enzymatique et/ou de fermentation. Le même effet est observé si l'hydrolyse enzymatique est conduite à forte teneur en matière sèche, la matière sèche d'un échantillon, exprimée en pourcentage poids, étant le ratio de masse de l'échantillon obtenu après séchage à 105°C pendant 24 heures sur la masse initiale de l'échantillon. Ce résidu solide doit alors être retraité, classiquement par combustion pour produire de la vapeur et de l'électricité, ou par méthanisation pour produire un biogaz.

Au cours de la dernière décennie les principaux travaux se sont attachés à comprendre les mécanismes d'action des cellulases et d'expression des enzymes afin de faire excréter le cocktail enzymatique le plus approprié à l'hydrolyse des substrats ligno-cellulosiques en modifiant les souches avec les outils de biologie moléculaire.

Le cocktail enzymatique est un mélange d'enzymes cellulolytiques et/ou hémicellulolytiques. Les enzymes du cocktail enzymatique contiennent trois grands types d'activités : les endoglucanases, les exoglucanases et les cellobiases, ces dernières étant également appelées β glucosidases.

Le microorganisme le plus utilisé pour la production du cocktail enzymatique est le champignon *Trichoderma reesei.* Les souches sauvages ont la faculté d'excréter, en présence d'un substrat inducteur, la cellulose par exemple, le cocktail enzymatique considéré comme le mieux adapté à l'hydrolyse de la cellulose. D'autres protéines possédant des propriétés indispensables à l'hydrolyse des matériaux ligno-cellulosiques sont également produites par *Trichoderma reesei,* les xylanases par exemple. La présence d'un substrat inducteur est indispensable à l'expression des enzymes cellulolytiques et/ou hémicellulolytiques. La nature du substrat carboné inducteur a une forte influence sur la composition du cocktail enzymatique. C'est le cas du xylose, qui, associé à un substrat carboné inducteur comme la cellulose ou le lactose, permet d'améliorer significativement l'activité dite xylanase.

Le lactose reste, dans un procédé de production industriel de cocktail enzymatique, un des substrats les plus appropriés; son coût varie cependant de manière importante et représente environ de un à deux tiers du prix de revient des enzymes. Dans le cas de l'utilisation de lactose comme source de carbone, le procédé de production de cocktail enzymatique est dépendant d'une source de carbone extérieure. De ce fait l'utilisation de substrats carbonés issus du procédé de conversion biochimique de matériaux ligno-cellulosiques est une voie de progrès importante si la source de carbone inductrice est facilement disponible.

La demande de brevet EP1690944 A1 enseigne l'utilisation de la phase aqueuse obtenue après fermentation alcoolique et séparation de l'éthanol comme source de carbone inductrice et de croissance pour la culture du microorganisme cellulolytique et la production d'enzymes. Le résidu obtenu dans le bas de la colonne de distillation est appelé "vinasses". Il est filtré et la partie soluble est utilisée pour la production des cellulases

La demande de brevet WO09026716 A1 décrit la production d'un cocktail enzymatique à partir de *Trichoderma reesei* à partir d'un substrat carboné contenant des sucres inducteurs de la production de cellulases. Cette demande enseigne que 3% poids de sucres inducteurs sont suffisant pour induire la production de cellulases. Les sucres inducteurs décrits sont des mono-, di- et oligo-saccharides produits éventuellement par l'hydrolyse de la cellulose.

La demande de brevet WO11028554 enseigne l'utilisation d'une biomasse ayant subi un traitement acide pour la culture d'un microbe permettant la production de cellulases, ainsi que l'utilisation du résidu solide issu de l'hydrolyse des hémicelluloses. Ce résidu solide est préalablement débarrassé de sa fraction lignine dans une étape d'extraction de la lignine. Le résidu solide délignifié est utilisé dès la phase de croissance du microorganisme, ce qui induit des difficultés d'opération, car la viscosité du milieu est forte tant que la production d'enzymes n'est pas démarrée.

Il est connu que la lignine, et notamment les composés phénolés qui la composent, a un effet inhibiteur sur les enzymes (voir par exemple « Inhibition of enzymatic cellulolysis by phenolic compounds », Tejirian et Xu, Enzyme and Microbial Technology vol.48(3) pp.239-247, Mars 2011).

Le brevet US3972775 enseigne la production de microorganisme cellulolytique et d'enzymes à partir de jus sucrés issus de l'hydrolysat et d'un inducteur cellulosique (par exemple de la charge cellulosique broyée). La charge et l'inducteur cellulosique sont des déchets cellulosiques, ne contenant donc pas de lignine.

Le brevet GB1489145 enseigne la culture du microorganisme cellulolytique et la production d'enzymes à partir de résidus de cellulose, ces derniers étant issus de matériaux cellulosiques purs ou de déchets cellulosiques. Ils ne proviennent donc pas directement du procédé de conversion du matériau ligno-cellulosique.

L'article de Prachand Shrestha et al. dans Journal of Agricultural and Food Chemistry, vol. 57, no. 10, pages 4156-4161 décrit un procédé de production d'un cocktail enzymatique à partir de *Trichoderma reesei* avec comme substrat des fibres de maïs provenant d'une stérilisation.

Un objet de l'invention est de proposer une source de carbone inductrice facilement disponible, permettant de produire un cocktail enzymatique aux activités appropriées à l'hydrolyse du matériau ligno-cellulosique. Cette invention permet par ailleurs la valorisation en interne de coproduits non valorisables en éthanol.

### RÉSUMÉ ET INTÉRÊT DE L'INVENTION

La présente invention concerne un procédé de production d'un cocktail enzymatique par un microorganisme cellulolytique caractérisé en ce qu'il utilise un résidu solide de l'hydrolyse enzymatique de matériaux ligno-cellulosiques ayant subit une étape de prétraitement et éventuellement un résidu solide de la fermentation éthanolique d'hydrolysats enzymatiques desdits matériaux.

Un avantage de l'invention est de réduire, voire de supprimer l'apport en substrat carboné d'origine externe au procédé de conversion biochimique de matériaux ligno-cellulosiques. Un autre avantage est de valoriser les résidus solides dudit procédé de conversion biochimique pour la production d'un cocktail enzymatique. Cette valorisation permet de réduire la quantité d'effluents produits qui doivent être retraités avant rejet ou stockage.

Lesdits résidus solides contenant de la cellulose et de la lignine étant valorisés pour la production d'un cocktail enzymatique, le coût dudit cocktail est réduit. Par ailleurs, lesdits résidus trouvant une voie de valorisation grâce au procédé selon l'invention, on peut se contenter d'une hydrolyse partielle dans l'étape d'hydrolyse enzymatique, obtenue en réduisant la quantité de cocktail enzymatique utilisée dans ladite étape d'hydrolyse. Ce dernier effet aboutit également à une réduction du coût du procédé de conversion biochimique du matériau ligno-cellulosique.

L'invention sera d'autant plus avantageuse que les matériaux ligno-cellulosiques traités seront réfractaires à l'hydrolyse enzymatique (peuplier, miscanthus par exemple).

Un avantage supplémentaire du procédé selon l'invention est de produire un cocktail enzymatique particulièrement adapté à l'hydrolyse enzymatique du matériau ligno-cellulosique prétraité converti dans le procédé de conversion biochimique. Lesdits résidus, bien que présentant une teneur élevée en lignine, ont de manière surprenante un effet inducteur permettant la production dudit cocktail enzymatique.

### DESCRIPTION DÉTAILLÉE DE L'INVENTION

La présente invention concerne un procédé de production d'un cocktail enzymatique avec un microorganisme cellulolytique comprenant deux phases :
- une phase a) de croissance dudit microorganisme en présence d'au moins un substrat carboné de croissance en réacteur fermé, ladite phase de croissance étant réalisée avec une concentration en substrat carboné de croissance comprise entre 10 et 90 g/L
- une phase b) de production du cocktail enzymatique dans laquelle au moins un substrat carboné inducteur est alimenté, ledit substrat carboné inducteur étant au moins un résidu solide issu de l'étape d'hydrolyse enzymatique de matériaux ligno-cellulosiques ayant subit une étape de prétraitement, la part de lignine dans la partie solide dudit résidu solide est de 45 à 80% poids, la partie solide du résidu solide représentant entre 10 et 40% dudit résidu, avec éventuellement au moins un résidu solide issu de l'étape de fermentation éthanolique d'hydrolysats enzymatiques, ladite phase de production étant réalisée avec une concentration en substrat carboné de production comprise entre 150 et 400 g/L.

Ledit procédé de production d'un cocktail enzymatique est mené en culture submergée. Par culture submergée, on entend une culture en milieu liquide.

Les microorganisme mis en oeuvre dans le procédé de production d'un cocktail enzymatique selon l'invention sont des souches de champignons appartenant aux genres *Trichoderma*, *Aspergillus*, *Penicillium* ou *Schizophyllum,* de préférence appartenant à l'espèce *Trichoderma reesei.* Les souches industrielles les plus performantes sont les souches appartenant à l'espèce *Trichoderma reesei,* modifiées pour améliorer le cocktail enzymatique par les procédés de mutation-sélection, comme par exemple la souche IFP CL847 (brevet français FR-B-2 555 803). Les souches améliorées par les techniques de recombinaison génétique peuvent être également utilisées. Ces souches sont cultivées en réacteurs agités et aérés dans des conditions compatibles avec leur croissance et la production des enzymes.

Le substrat carboné de croissance dudit microorganisme utilisé dans ladite phase a) de croissance du procédé selon l'invention est avantageusement choisi parmi les sucres solubles industriels, et de préférence parmi le glucose, le lactose, le xylose, les résidus liquides obtenus après fermentation éthanolique des sucres monomères des hydrolysats enzymatiques de matériaux ligno-cellulosique et les extraits de la fraction hémicellulosique sous forme de monomères provenant de substrat lignocellulosique prétraité, utilisé seul ou en mélange. Selon sa nature, ledit substrat carboné de croissance est introduit dans le réacteur fermé avant stérilisation ou est stérilisé séparément et introduit dans le réacteur fermé après stérilisation de ce dernier.

Conformément à l'invention, ledit substrat carboné de croissance est utilisé dans ladite phase a) de croissance à une concentration initiale comprise entre 10 et 90 g de substrat carboné par litre de volume réactionnel.

De préférence, ladite phase a) de croissance est réalisée sur une durée comprise entre 30 et 70 h, de préférence entre 30 et 40 h.

De préférence, ladite phase a) de croissance opère à un pH de 4,8 et à une température de 27°C.

Conformément à l'invention, ledit substrat carboné inducteur utilisé dans ladite phase b) de production est avantageusement au moins un résidu solide issu de l'étape d'hydrolyse enzymatique de matériaux ligno-cellulosiques ayant subit une étape de prétraitement et/ou au moins un résidu solide issu de l'étape de fermentation éthanolique d'hydrolysats enzymatiques.

Ledit résidu solide est de préférence obtenu après une hydrolyse enzymatique partielle, c'est à dire lorsque cette dernière est opérée à forte teneur en matière sèche (MS) et/ou à faible quantité de cocktail enzymatique. Par forte teneur en MS, on entend supérieure à 20% poids de MS. Par faible quantité de cocktail enzymatique, on entend inférieure à 10 mg de cocktail enzymatique par g de cellulose dans le matériau ligno-cellulosique ayant subit une étape de prétraitement. Par hydrolyse partielle, on entend que seul 20 à 70% poids de la cellulose en entrée de l'étape d'hydrolyse est hydrolysé.

Ledit résidu solide est avantageusement séparé de l'effluent de l'étape d'hydrolyse enzymatique et, lorsqu'il est utilisé, de l'effluent de l'étape de fermentation éthanolique. La séparation est avantageusement réalisée par filtration, centrifugation ou toute autre méthode connue de l'homme du métier permettant la séparation d'une phase solide et d'une phase liquide.

Ledit résidu solide est donc directement issu du procédé de conversion biochimique de matériaux ligno-cellulosiques.

Ledit résidu solide comprend une partie solide et une partie liquide. En fonction de la méthode de séparation utilisée, la partie solide du résidu solide représente entre 10 et 40% du poids du résidu solide. Ladite partie solide est constituée de lignine, de composés minéraux et de cellulose non hydrolysée. La part de cellulose dans ladite partie solide est de 10 à 50% poids. La part de lignine dans ladite partie solide est de 45 à 80% poids. La part des composés minéraux dans ladite partie solide est de 1 à 15% poids. La part liquide dudit résidu solide contient du xylose (non fermenté par la levure *Saccharomyces cerevisiae*) qui est inducteur de la production de xylanases.

De préférence, ledit substrat carboné inducteur est utilisé en mélange avec au moins un autre substrat carboné.

De préférence, ledit autre substrat carboné est choisi parmi des sucres inducteurs ou non inducteurs, de manière préférée choisi parmi le lactose, le glucose, l'hydrolysat cellulosique, l'hydrolysat hémicellulosique, le cellobiose et le xylose, pris seul ou en mélange. De manière très préférée, ledit autre substrat carboné est choisi parmi des sucres non inducteurs, de manière très préférée parmi le glucose, l'hydrolysat cellulosique et l'hydrolysat hémicellulosique.

On appelle ce mélange substrat carboné de production. Ledit substrat carboné de production contient au moins 5% poids de cellulose.

Le substrat carboné de production est préparée à la concentration de 150 à 400 g de substrat carboné par litre de substrat carboné de production. Le débit spécifique de l'alimentation en substrat carboné de production de la phase b) de production est avantageusement compris entre 35 et 65 mg de substrat carboné par gramme de microorganisme et par heure, préférentiellement de 35 à 45 mg de substrat carboné par gramme de microorganisme et par heure.

Ledit résidu solide est issu de l'étape d'hydrolyse enzymatique de matériaux ligno-cellulosiques ayant subit une étape de prétraitement . Eventuellement il est ajouté un résidu solide issu de l'étape de fermentation éthanolique d'hydrolysats enzymatiques.

L'étape de prétraitement du matériau ligno-cellulosique permet d'améliorer la susceptibilité à l'hydrolyse enzymatique de la fraction cellulosique. De préférence, l'étape de prétraitement est une étape de prétraitement acide, de préférence une hydrolyse acide, une cuisson acide ou une explosion à la vapeur avec imprégnation préalable dudit matériau avec une solution aqueuse d'acide sulfurique. De manière préférée, l'étape de prétraitement est l'explosion à la vapeur.

À l'issue de l'étape de prétraitement, un résidu solide peut avantageusement être séparé d'une fraction liquide contenant des sucres, appelée hydrolysat hémicellulosique, par séparation liquide/solide. Ledit résidu peut également avantageusement être utilisé dans la phase b) de production du cocktail enzymatique selon l'invention comme substrat carboné inducteur. Le substrat carboné inducteur utilisé dans la phase b) de production peut donc avantageusement être séparé à l'issu de l'étape de prétraitement d'un matériau ligno-cellulosique.

De préférence, ladite phase b) de production est réalisée sur une durée au moins supérieure à 30 h, de préférence au moins supérieure à 100 h.

De préférence, ladite phase b) de production opère à un pH compris entre 3 et 5,5 et à une température de comprise entre 20 et 30°C.

Ladite phase b) de production peut être conduite selon les modes fed-batch et chemostat selon la terminologie anglo-saxonne, connus de l'homme du métier.

Dans un mode de réalisation préféré, le matériau ligno-cellulosique prétraité est hydrolysé dans une étape d'hydrolyse enzymatique. L'effluent de cette étape est ensuite traité dans une étape de fermentation éthanolique des sucres monomères des hydrolysats enzymatiques. Ces traitements peuvent être réalisés dans le même équipement, ou dans des équipements différents. Le résidu solide est séparé après l'étape d'hydrolyse enzymatique et/ou après l'étape de fermentation éthanolique.

Dans un autre mode de réalisation préféré, l'étape de fermentation et au moins une partie de l'étape d'hydrolyse sont réalisées simultanément. Ceci est réalisé, par exemple, en ajoutant des levures éthanoliques au cours de l'étape d'hydrolyse. Le résidu solide est séparé à l'issue de l'étape de fermentation.

L'étape d'hydrolyse est démarrée par ajout du cocktail enzymatique. La quantité avantageusement utilisée est de 1 à 60 mg de cocktail enzymatique par gramme de cellulose dans le matériau ligno-cellulosique ayant subit une étape de prétraitement, de préférence de 5 à 35 mg de cocktail enzymatique par gramme de cellulose dans ledit matériau, et plus préférentiellement de 5 à 20 mg de cocktail enzymatique par gramme de cellulose dans ledit matériau. Ladite étape d'hydrolyse est mise en oeuvre avec de 10% à 40% poids de MS, préférentiellement de 15 à 25% poids de MS.

Les exemples qui suivent illustrent l'invention sans en limiter la portée.

### Exemple 1: Production d'un cocktail enzymatique sur glucose (non conforme)

La production d'un cocktail enzymatique est effectuée en réacteur agité mécaniquement. Le milieu minéral (dit 4N) a la composition suivante : KOH 1,66 g/L, H₃PO₄ 85 % 2 mL/L, (NH₄)₂SO₄ 2,8 g/L, MgSO₄, 7 H₂O 0,6 g/L, CaCL₂ 0,6 g/L, MnSO₄ 3,2 mg/L, ZnSO₄, 7 H₂O 2,8 mg/L, CoCl₂ 10 4,0 mg/L, FeSO₄, 7 H₂O 10 mg/L, Corn Steep 1,2 g/L, anti-mousse 0,5 mL/L

### Préculture liquide

La croissance du microorganisme (la souche de *Trichoderma reesei* CL847) en préculture est faite en utilisant le glucose comme substrat carboné de croissance, à la concentration de 30 g/L. Le milieu minéral de la préculture est le milieu 4N additionné de phtalate de potassium à 5 g.L⁻¹ afin de tamponner le pH. La croissance de l'inoculum dure de 3 jours et est effectuée à 30 °C dans un incubateur agité. Le transfert vers le réacteur est réalisé si la concentration résiduelle en glucose est inférieure à 15 g/L

### Phase de croissance

Le réacteur contenant le milieu 4N est stérilisé à 120 °C pendant 20 minutes. Le substrat carboné de croissance glucose est stérilisé à part à 120°C pendant 20 minutes puis ajouté stérilement dans le réacteur de façon à avoir une concentration de 30 g/L. Le réacteur est ensemencé à 10% (v/v) avec la préculture liquide de la souche de *Trichoderma reesei* CL847. Les conditions opératoires sont une température de 27 °C et un pH de 4,8 (régulé par de l'ammoniaque 5.5 M). L'aération est de 0,5 vvm et l'agitation est augmentée entre 200 et 800 rpm en fonction de la pO₂ (pression en oxygène dissous), qui est maintenue à 30 %.

### Phase de production

Lorsque le substrat carboné de croissance du réacteur est épuisé, le substrat carboné de production glucose à 250 g/L est injecté en continu au débit de 35 mg par g de microorganisme et par heure jusqu'à 164 heures. Les conditions opératoires sont une température de 25 °C et un pH de 4 (régulé par de l'ammoniaque 5.5 M, ce dernier apportant également l'azote nécessaire à la synthèse des protéines excrétées). La teneur en oxygène dissous est maintenue à 30% par action sur l'agitation.

La production du cocktail enzymatique est suivie par le dosage des protéines extracellulaires par la méthode de Lowry et standard BSA, après séparation du mycélium par filtration ou centrifugation.

Les déterminations analytiques sur le moût final donnent les résultats suivants :

| | |
|---|---|
| • Biomasse (g/L) | 15.2 |
| • Protéines (g/L) | 2.9 |
| • qₚ (mg/g/h) | 1.2 |

qₚ étant la vitesse spécifique de production de cocktail enzymatique.

### Exemple 2 : Production d'enzymes sur lactose (non conforme)

La production du cocktail enzymatique est menée dans les mêmes conditions que dans l'Exemple 1. Le substrat carboné de croissance et de production est le lactose pur. Le lactose est un inducteur important de la production du cocktail enzymatique.

Après 30 heures de croissance, après l'épuisement du substrat carboné de croissance, la solution de fed-batch à 250 g/L est injectée en continu au débit de 35 mg par g de microorganisme et par heure jusqu'à 164 heures.

Les déterminations analytiques sur le moût final donnent les résultats suivants :

| | |
|---|---|
| • Biomasse (g/L) | 13.5 |
| • Protéines (g/L) | 37.8 |
| • qₚ (mg/g/h) | 17 |

L'induction de la production du cocktail enzymatique par le lactose est clairement mis en évidence (concentration en protéines et fort qₚ).

### Exemple 3 : Effet inducteur du résidu solide et de différents substrats carbonés

L'étude de l'effet inducteur de différents substrats carbonés est réalisé en fioles à partir d'une même préculture. Les fioles sont réalisées en doublons et les substrats carbonés de production utilisés sont: Le glucose (répresseur de la production du cocktail enzymatique), le lactose (inducteur), la cellulose (inducteur), le résidu solide d'hydrolyse à deux concentrations différentes. Le résidu solide est issu d'une hydrolyse partielle (70%) d'une paille de blé prétraitée par explosion à la vapeur en conditions acides. Ce résidu a été lavé et pressé à 30 % de MS. Sa composition est la suivante: 19 % de cellulose, 59% de lignine, 2.9% d'hémicelluloses, 0.6% acétyls, 11% de cendres, 7.5% nd (non déterminé)

La croissance du champignon *T.reesei* en préculture est faite sur glucose, à la concentration de 10 g.L⁻¹. La croissance de l'inoculum dure 3 jours et est effectuée à 30 °C dans un incubateur infors sous agitation (150 rpm) dans deux fioles de Fernbach avec un volume utile de 350 mL. Les deux fioles sont mélangées à la fin de la préculture. La concentration résiduelle en glucose est de 0.3 g/L.

La composition des fioles est détaillée dans le tableau 1. Elles sont stérilisées avant ensemencement:

| **Composition Fioles** | témoin | **résidu solide (1g)** | | **résidu solide (4g)** | | glucose | | lactose | | cellulose | |
|---|---|---|---|---|---|---|---|---|---|---|---|
| | Fiole **0** | **Fiole 1** | **Fiole 2** | **Fiole 3** | **Fiole 4** | **Fiole 5** | **Fiole 6** | **Fiole 7** | **Fiole 8** | **Fiole 9** | **Fiole 10** |
| Milieu salin 4N (mL) | 25 | 25 | 25 | 25 | 25 | 25 | 25 | 25 | 25 | 25 | 25 |
| Phtalate de potassium(g) | 0,6 | 0,6 | 0,6 | 0,6 | 0,6 | 0,6 | 0,6 | 0,6 | 0,6 | 0,6 | 0,6 |
| Extrait de levure(g) | 0.1 | 0.1 | 0.1 | 0.1 | 0.1 | 0.1 | 0.1 | 0.1 | 0.1 | 0.1 | 0.1 |
| Matières issues de l'hydrolyse H10 (g) | 0 | **1** | **1** | **4** | **4** | 0 | 0 | 0 | 0 | 0 | 0 |
| Glucose à 200g/L (mL) | 0 | 0 | 0 | 0 | 0 | **5** | **5** | 0 | 0 | 0 | 0 |
| Lactose à 200g/L (mL) | 0 | 0 | 0 | 0 | 0 | 0 | 0 | **5** | **5** | 0 | 0 |
| Cellulose : Nutrafiber (g) | 0 | 0 | 0 | 0 | 0 | 0 | 0 | 0 | 0 | **1** | **1** |
| Eau(mL) | 25 | 25 | 25 | 25 | 25 | 20 | 20 | 20 | 20 | 25 | 25 |
| Ensemencement(mL) | 50 | 50 | 50 | 50 | 50 | 50 | 50 | 50 | 50 | 50 | 50 |

Les fioles sont incubées dans un incubateur Infors à 30°C et sous agitation (150 rpm). Les déterminations analytiques sur le moût final (après 94 h) donnent les résultats suivants :

| | Concentration en Protéines | qp (mg/g/h) |
|---|---|---|
| Fiole 0 | 1,02 | 0,10 |
| Fiole 1 | 1,95 | 3,92 |
| Fiole 2 | 2,09 | 4,52 |
| Fiole 3 | 2,52 | 6,31 |
| Fiole 4 | 2,39 | 5,75 |
| Fiole 5 | 0,97 | -0,12 |
| Fiole 6 | 0,97 | -0,13 |
| Fiole 7 | 3,02 | 8,35 |
| Fiole 8 | 2,81 | 7,51 |
| Fiole 9 | 2,47 | 6,10 |
| Fiole 10 | 2,43 | 5,90 |

Le témoin et les fioles utilisant le glucose ont une concentration de 1 g/L qui correspond probablement à la concentration d'extrait de levure. Le qp moyen de ces fioles est proche de 0. La fiole avec le lactose aboutit à une concentration en protéines de 3 g/L. La concentration en protéines avec la cellulose et le résidu solide à 4 g est de 2.5 g/L. Elle est de 2 g/L avec 1 g de résidu solide. Cette expérience met en évidence l'induction par le résidu solide de la production du cocktail enzymatique, à un niveau comparable à la cellulose seule, et ce malgré la présence de lignine.

## Revendications

1. Un procédé de production d'un cocktail enzymatique avec un microorganisme cellulolytique comprenant deux phases :
- une phase a) de croissance dudit microorganisme en présence d'au moins un substrat carboné de croissance en réacteur fermé, ladite phase de croissance étant réalisée avec une concentration en substrat carboné de croissance comprise entre 10 et 90 g/L
- une phase b) de production du cocktail enzymatique dans laquelle au moins un substrat carboné inducteur est alimenté, ledit substrat carboné inducteur étant au moins un résidu solide issu de l'étape d'hydrolyse enzymatique de matériaux ligno-cellulosiques ayant subi une étape de prétraitement, la part de lignine dans la partie solide dudit résidu solide est de 45 à 80% poids, la partie solide du résidu solide représentant entre 10 et 40% dudit résidu, ladite phase de production étant réalisée avec une concentration en substrat carboné de production comprise entre 150 et 400 g/L.

2. Procédé selon la revendication 1 dans lequel ledit résidu solide est obtenu après une hydrolyse enzymatique partielle et 20 à 70% poids de la cellulose en entrée de l'étape d'hydrolyse est hydrolysé.

3. Procédé selon l'une des revendications 1 à 2 dans lequel la part de cellulose dans la partie solide du résidu solide est de 10-50% pds, la partie solide du résidu solide représentant entre 10 et 40% dudit résidu.

4. Procédé selon l'une des revendications 1 à 3 dans lequel ledit résidu solide est séparé de l'effluent de l'étape d'hydrolyse enzymatique.

5. Procédé selon l'une des revendications 1 à 4 dans lequel ledit substrat carboné inducteur est utilisé en mélange avec au moins un autre substrat carboné choisi parmi des sucres inducteurs ou non inducteurs.

6. Procédé selon la revendication 5 dans lequel ledit autre substrat carboné est choisi parmi le lactose, le glucose, l'hydrolysat cellulosique, l'hydrolysat hémicellulosique, le cellobiose et le xylose, pris seul ou en mélange.

7. Procédé selon l'une des revendications 1 à 6 dans lequel le substrat carboné de production contient au moins 5% poids de cellulose

8. Procédé selon l'une des revendications 1 à 7 dans lequel le débit spécifique de l'alimentation de substrat carboné de production utilisé dans ladite phase b) de production est compris entre 35 et 65 mg de substrat carboné par gramme de microorganisme et par heure.

9. Procédé selon l'une des revendications 1 à 8 dans lequel ladite étape d'hydrolyse est démarrée avec 1 à 60 mg de cocktail enzymatique par gramme de cellulose dans le matériau ligno-cellulosique ayant subi une étape de prétraitement.

10. Procédé selon la revendication 9 dans lequel ladite étape d'hydrolyse est mise en oeuvre avec de 10% à 40% poids de MS.

11. Procédé selon l'une des revendications 1 à 8 dans lequel le microorganisme cellulolytique est choisi parmi les souches de champignons appartenant aux genres *Trichoderma*, *Aspergillus*, *Penicillium* ou *Schizophyllum.*

12. Procédé selon la revendication 11 dans lequel le microorganisme cellulolytique appartient à l'espèce *Trichoderma reesei*

## Patentansprüche

1. Verfahren zur Herstellung eines Enzymcocktails mit einem cellulolytischen Mikroorganismus, umfassend zwei Phasen:
- eine Wachstumsphase a) des Mikroorganismus in Anwesenheit mindestens eines kohlenstoffhaltigen Wachstumssubstrats im geschlossenen Reaktor, wobei die Wachstumsphase mit einer Konzentration des kohlenstoffhaltigen Wachstumssubstrats zwischen 10 und 90 g/l durchgeführt wird,
- eine Produktionsphase b) des Enzymcocktails, wobei mindestens ein kohlenstoffhaltiges Induktorsubstrat zugeführt wird, wobei das kohlenstoffhaltige Induktorsubstrat mindestens ein fester Rückstand aus dem Enzymhydrolyseschritt von Lignocellulosematerialien ist, die einem Vorbehandlungsschritt unterzogen wurden, wobei der Ligninanteil in dem festen Anteil des festen Rückstands von 45 bis 80 % beträgt, wobei der feste Teil des festen Rückstands zwischen 10 und 40 % des Rückstands beträgt, wobei die Produktionsphase mit einer Konzentration des kohlenstoffhaltigen Produktionssubstrats zwischen 150 und 400 g/l durchgeführt wird.

2. Verfahren nach Anspruch 1, wobei der feste Rückstand nach einer partiellen Enzymhydrolyse erhalten wird, und 20 bis 70 Gew.-% der Cellulose am Eingang des Hydrolyseschritts hydrolysiert werden.

3. Verfahren nach einem der Ansprüche 1 bis 2, wobei der Celluloseanteil in dem festen Anteil des festen Rückstands von 10 bis 50 Gew.-% beträgt, wobei der feste Anteil des festen Rückstands zwischen 10 und 40 % des Rückstands beträgt.

4. Verfahren nach einem der Ansprüche 1 bis 3, wobei der feste Rückstand von dem Abfluss des Enzymhydrolyseschritts getrennt wird.

5. Verfahren nach einem der Ansprüche 1 bis 4, wobei das kohlenstoffhaltige Induktorsubstrat in Mischung mit mindestens einem anderen kohlenstoffhaltigen Substrat verwendet wird, ausgewählt aus Induktor- oder Nicht-Induktor-Zuckern.

6. Verfahren nach Anspruch 5, wobei das andere kohlenstoffhaltige Substrat ausgewählt wird aus Lactose, Glucose, Cellulosehydrolysat, Hemicellulosehydrolysat, Cellobiose und Xylose, allein oder in Mischung.

7. Verfahren nach einem der Ansprüche 1 bis 6, wobei das kohlenstoffhaltige Produktionssubstrat mindestens 5 Gew.-% Cellulose enthält.

8. Verfahren nach einem der Ansprüche 1 bis 7, wobei der spezifische Durchsatz der Zufuhr des kohlenstoffhaltigen Produktionssubstrats, das in der Phase b) verwendet wird, zwischen 35 und 65 mg kohlenstoffhaltiges Substrat pro Gramm Mikroorganismus und pro Stunde beträgt.

9. Verfahren nach einem der Ansprüche 1 bis 8, wobei der Hydrolyseschritt mit 1 bis 60 mg Enzymcocktail pro Gramm Cellulose in dem Lignocellulosematerial gestartet wird, das einem Vorbehandlungsschritt unterzogen wurde.

10. Verfahren nach Anspruch 9, wobei der Hydrolyseschritt mit 10 Gew.-% bis 40 Gew.-% MS durchgeführt wird.

11. Verfahren nach einem der Ansprüche 1 bis 8, wobei der cellulolytische Mikroorganismus ausgewählt wird aus Pilzstämmen, die zu den Gattungen *Trichoderma*, *Aspergillus*, *Penicillium* oder *Schizophyllum* gehören.

12. Verfahren nach Anspruch 11, wobei der cellulolytische Mikroorganismus zur Art *Trichoderma reesei* gehört.

## Claims

1. A process for the production of an enzymatic cocktail with a cellulolytic microorganism, comprising two phases:
• a phase a) for growth of said microorganism in the presence of at least one carbonaceous growth substrate in a closed reactor, said growth phase being carried out with a concentration of carbonaceous growth substrate in the range 10 to 90 g/L;
• a phase b) for the production of the enzymatic cocktail, in which at least one carbonaceous inducer substrate is supplied, said carbonaceous inducer substrate being at least one solid residue obtained from the step for enzymatic hydrolysis of lignocellulosic materials which have undergone a pre-treatment step, the portion of lignin in the solid portion of said solid residue is 45% to 80% by weight, the solid portion of the solid residue representing between 10 and 40% of said residue, said production phase being carried out with a concentration of carbonaceous production substrate in the range 150 to 400 g/L.

2. The process according to claim 1, in which said solid residue is obtained after a partial enzymatic hydrolysis, and 20 to 70% by weight of cellulose at the inlet of the hydrolysis step is hydrolysed..

3. The process according to claim 1 or 2, in which the portion of cellulose in the solid portion of said solid residue is 10% to 50% by weight, the solid portion of the solide residue representning 10 to 40% of said residue.

4. The process according to one of claims 1 to 3, in which said solid residue is separated from the effluent of the enzymatic hydrolysis step.

5. The process according to one of claims 1 to 4, in which said carbonaceous inducer substrate is used as a mixture with at least one other carbonaceous substrate selected from inducer or non-inducer sugars.

6. The process according to claim 5, in which said other carbonaceous substrate is selected from lactose, glucose, cellulosic hydrolysate, hemicellulosic hydrolysate, cellobiose and xylose, used alone or as a mixture.

7. The process according to one of claims 1 to 6, in which the carbonaceous production substrate contains at least 5% by weight of cellulose.

8. The process according to one of claims 1 to 7, in which the specific flow rate at which the carbonaceous production substrate used in said production phase b) is supplied is in the range 35 to 65 mg of carbonaceous substrate per gram of microorganism per hour.

9. The process according to one of claims 1 to 8, in which said hydrolysis step is initiated with 1 to 60 mg of enzymatic cocktail per gram of cellulose in the lignocellulosic material which has undergone a pre-treatment step.

10. The process according to claim 9, in which said hydrolysis step is carried out with 10% to 40% by weight of DM.

11. The process according to one of claims 1 to8, in which the cellulolytic microorganism is selected from strains of fungi belonging to the genuses *Trichoderma, Aspergillus, Penicillium* or *Schizophyllum.*

12. The process according to claim 12, in which the cellulolytic microorganism belongs to the species *Trichoderma reesei.*
